# EUROPEAN PATENT APPLICATION

(11) **EP 3 318 240 A1**
(43) Date of publication of application: **09.05.2018**
(21) Application number: 16197785.5
(22) Date of filing: 08.11.2016
(51) Int. Cl.: A61H 3/00, A47C 9/02, A61F 5/01

(54) **WEARABLE SITTING POSTURE ASSISTING DEVICE AND METHOD FOR PUTTING ON SUCH A DEVICE**

(71) Applicant: noonee AG, 8630 Rüti ZH (CH)
(72) Inventor: GUNURA, Keith, 8003 Zürich (CH); VAFI, Daniel, 8006 Zürich (CH); ANASTASIADES, Bryan, 8046 Zürich (CH); JERGEN, Robin, 8134 Adliswil (CH); HUTTER, Simon, 9434 Au (CH); HUWYLER, Willy, 6330 Cham (CH); GEHRING, Alexandra, 8182 Hochfelden (CH)
(74) Representative: Daub, Thomas

(57) **Abstract**

The invention is based on a wearable sitting posture assisting device (100a), comprising at least one leg unit (102a) featuring at least one upper leg (106a) and at least one lower leg (124a; 124b; 124c), the lower leg (124a; 124b; 124c) defining at least one lower leg longitudinal axis (128a; 128b; 128c; 128f); and comprising at least one foot unit (142a; 142b; 142c; 142e; 142f; 142g) having at least one shoe connector (144a; 144b; 144e; 144f; 144g) for connecting to a shoe (214a) or a foot of a person (200a).

It is proposed that the shoe connector (144a; 144b; 144e; 144f; 144g) is movable relative to the lower leg (124a; 124b; 124c) in a direction (10a; 10b) at least substantially parallel to the lower leg longitudinal axis (128a; 128b; 128c; 128f) in at least one shoe connector wearing state.

## Description

### State of the Art

The invention relates to a wearable sitting posture assisting device.

A posture assisting device is known from the document WO 2015/028373 A1.

The objective of the invention is, in particular, to provide a generic wearable sitting posture assisting device with improved characteristics regarding comfort. The objective is achieved according to the invention by the features of patent claims 1 and 17, while advantageous embodiments and further developments of the invention may be gathered from the dependent claims.

### Advantages of the Invention

The invention relates to a wearable sitting posture assisting device, comprising at least one leg unit featuring at least one upper leg and at least one lower leg, the lower leg defining at least one lower leg longitudinal axis; and comprising at least one foot unit having at least one shoe connector for connecting to a shoe or a foot of a person.

It is proposed that the shoe connector is movable relative to the lower leg in a direction at least substantially parallel to the lower leg longitudinal axis in at least one shoe connector wearing state.

By means of the invention a high degree of comfort can be achieved. Furthermore, user-friendliness can be improved. Advantageously, a wearable sitting posture assisting device can be provided which is easy to handle. A high degree of comfort and/or user-friendliness during putting a wearable sitting posture assisting device on and/or off can be achieved. Furthermore, easy and/or comfortable and/or fast individualization of components is facilitated. In addition, a high degree of wearing comfort of a wearable sitting posture assisting device can be achieved.

A "wearable sitting posture assisting device" is herein to be understood as a device which is configured for receiving a weight force of a person in a sitting posture or in a partly sitting posture and to transmit the weight force to a ground. In particular, an angle between a thigh and a shank of the person in the sitting posture is no greater than 130°, preferably no greater than 120° and advantageously no greater than 110° and/or no smaller than 60°, preferably no smaller than 70° and advantageously no smaller than 80°. In particular, the angle between the thigh and the shank of the person is approximately 90° in the sitting posture. In particular, an angle between a thigh and a shank of the person in the partly sitting posture is no greater than 170°, preferably no greater than 160° and advantageously no greater than 150° and/or no smaller than 100°, preferably no smaller than 110° and advantageously no smaller than 120°. In particular, the angle between the thigh and the shank of the person is approximately 130° in the partly sitting posture. It is conceivable that the partly sitting posture is a posture in which the person is leaning forward while partly bending his knees. In particular, a person wearing the wearable sitting posture assisting device is enabled to sit and/or to partly sit and/or to take a seat on the wearable sitting posture assisting device, wherein at least a part of the weight force is counteracted by the wearable sitting posture assisting device and/or wherein the person is counteracting only a fraction of the weight force using his muscles. In particular, the wearable sitting posture assisting device is configured for being worn by the person while the person is standing and/or while the person is walking. Advantageously, the wearable sitting posture assisting device is configured for supporting different sitting postures and/or partly sitting postures, which are in particular characterized by different sitting angles.

The wearable sitting posture assisting device in particular defines a sitting direction. Preferably, the person faces and/or looks in the sitting direction when sitting or partly sitting on the wearable sitting posture assisting device and facing forward. In particular, the sitting direction is oriented parallel to a floor on which the person is standing and/or sitting and/or walking when wearing the wearable sitting posture assisting device. In particular, the wearable sitting posture assisting device defines a walking direction. Preferably, the person faces in the walking direction when walking and/or standing with the wearable sitting posture assisting device and facing forward. In particular, the walking direction is oriented parallel to a floor on which the person is standing and/or sitting and/or walking when wearing the wearable sitting posture assisting device. In particular, the wearable sitting posture assisting device is only designed to receive and transmit the weight force. Preferably, the wearable sitting posture assisting device is not designed to generate a controllable force, which is provided to assist a person while walking, standing or lifting some loads. In this context, "configured" is in particular to mean specifically programmed, designed and/or equipped. By an object being configured for a certain function is in particular to be understood that the object implements and/or fulfills said certain function in at least one application state and/or operating state.

Preferably, the wearable sitting posture assisting device comprises at least one leg unit. In particular, the wearable sitting posture assisting device comprises at least one additional leg unit. In particular, the leg unit comprises at least one, preferably one, upper leg and/or at least one, preferably one, lower leg and/or at least one, preferably one, foot unit and/or at least one, preferably one, ground contact unit. Advantageously, the upper leg is connected to the lower leg, in particular via at least one, in particular one, knee joint. Preferably, the foot unit is connected to the lower leg. Advantageously, the ground contact unit is connected to the lower leg and/or to the foot unit. It is conceivable that the ground contact unit is at least partly implemented integrally with the lower leg and/or at least partly implemented integrally with the foot unit. It is also conceivable that the foot unit is at least partly implemented integrally with the lower leg. Preferably, the wearable sitting posture assisting device comprises at least one upper body wearing unit. In particular, the leg unit is connected to the upper body wearing unit, preferably via at least one connection strap. In this context, the term "a first object and a second object being at least partly implemented integrally" is in particular to mean that at least one component of the first object and at least one component of the second object are implemented integrally with each other. "Implemented integrally" is in particular to mean, in this context, connected at least by substance-to-substance bond, e.g., by a welding process, an adhesive bonding, an injection-molding process and/or by another process that is deemed expedient by a person having ordinary skill in the art. Advantageously, "implemented integrally" could in particular mean made of one piece. "Made of one piece" is, in particular, to mean, in this context, manufactured from one single piece, e.g., by production from one single cast and/or by manufacturing in a one-component or multi-component injection-molding process, and advantageously from a single blank.

Preferably, the wearable sitting posture assisting device comprises two leg units. Advantageously, the leg unit and the additional leg unit are implemented identically. It is also conceivable that the leg unit and the additional leg unit are implemented mirror-symmetrically with respect to each other. It is conceivable that the leg unit is configured for being worn on a left leg and the additional leg unit is configured for being worn at a right leg, or vice versa. Advantageously, the leg unit is configured for being worn either on a left leg or on a right leg. Further advantageously, the additional leg unit is configured for being worn on a left leg or on a right leg. Preferably, the additional leg unit is connected to the upper body wearing unit, preferably via at least one connection strap. In particular, the person wearing the wearable sitting posture assisting device is wearing the leg unit, in particular solely, on a first leg, for instance a left leg or a right leg. In particular, the person wearing the wearable sitting posture assisting device is wearing the additional leg unit, in particular solely, on a second leg, for instance a right leg or a left leg. Advantageously, the leg unit is arranged on a rear side of the leg on which the leg unit is worn. Further advantageously, the additional leg unit is arranged on a read side of the leg on which the additional leg unit is worn. In particular, the leg units of the wearable sitting posture assisting device are arranged on a rear side of the legs of the person when the person is sitting and/or partly sitting on the wearable sitting posture assisting device and/or standing and/or walking with the wearable sitting posture assisting device. Preferably, the person wearing the wearable sitting posture assisting device is wearing the upper body wearing unit on his upper body. Advantageously, the upper body wearing unit is implemented as a belt and/or as braces and/or as suspenders. The wearable sitting posture assisting device enables the person wearing it to walk around, stand, take a seat on the wearable sitting posture assisting device if required or wanted and stand up after sitting or partly sitting on the wearable sitting posture assisting device.

Preferably, the upper leg comprises at least one thigh connection unit for connecting to a thigh of the person. Preferably, the thigh connection unit features at least one thigh strap. In particular, the upper leg comprises a seat unit configured for providing at least one sitting surface for the person, in particular in case the person is sitting or partly sitting on the wearable sitting posture assisting device, preferably for the thigh and/or at least a lower portion of a buttock of the person, wherein "buttock" is preferably to mean one cheek of the buttocks. Preferably, the seat unit comprises at least one sitting element which features the sitting surface. Advantageously, the seat unit is in contact with the thigh of the person in case the person is sitting or partly sitting on the wearable sitting posture assisting device. Preferably, the seat unit is arranged on a rear side of the thigh of the person in case the person is standing or walking with the wearable sitting posture assisting device.

Advantageously, the upper leg comprises at least one upper leg support. Preferably, the seat unit is connected to the upper leg support. Advantageously, the thigh connection unit and/or the thigh strap is connected to the upper leg support. In particular, the upper leg support is implemented as a frame element. Preferably, the upper leg support is implemented as an elongated element.

Advantageously, the upper leg features at least one upper leg longitudinal axis which is oriented at least substantially parallel to a longitudinal axis of the thigh of the person. Preferably, a main extension direction of the upper leg support is oriented at least substantially parallel, or parallel, to the upper leg longitudinal axis. In particular, the upper leg support is at least partly, preferably at least to a large extent, advantageously completely made of plastic. It is also conceivable that the upper leg support is at least partly, preferably at least to a large extent, advantageously completely made of metal, in particular made of a light metal or a light alloy, for instance aluminum and/or titanium and/or beryllium and/or scandium or other suitable metals. It is further conceivable that the upper leg support is at least partly, preferably at least to a large extent, advantageously completely made of a composite material, in particular a fiber reinforced composite material and/or a fiber reinforced plastic and/or a carbon fiber reinforced material and/or a carbon fiber reinforced polymer and/or a fiber reinforced thermoplastic. The term "at least to a large extent" is in particular to mean to an extent of at least 55 %, preferably to an extent of at least 65 %, further preferably to an extent of at least 75 %, advantageously to an extent of at least 85 % and further advantageously to an extent of at least 95 %. In this context "at least substantially parallel" is in particular to be understood as an orientation of a direction with respect to a reference direction, in particular in a plane, wherein the direction has a deviation from the reference direction in particular of less than 15º, advantageously of less than 10º and particularly advantageously of less than 2º. A "main extension direction" of an object is, in particular, to be understood, in this context, as a direction extending in parallel to a largest side of an imaginary rectangular cuboid which only just entirely encloses the object.

Preferably, the lower leg is arranged at a rear side of the shank of the person. In particular, the lower leg comprises at least one lower leg support. Advantageously, the lower leg support is implemented as a frame element. Preferably, the lower leg support is implemented as an elongated element. In particular, the lower leg features at least one lower leg longitudinal axis which is oriented at least substantially parallel to a longitudinal axis of the shank of the person. Preferably, a main extension direction of the lower leg support is oriented at least substantially parallel, or parallel, to the lower leg longitudinal axis. Advantageously, the lower leg longitudinal axis is oriented at least substantially parallel, or parallel, to the upper leg longitudinal axis.

Preferably, the upper leg and the lower leg together define a sitting angle. Advantageously, the sitting angle is an angle included between the upper leg longitudinal axis and the lower leg longitudinal axis, in particular on a rear side of the upper leg and the lower leg. In particular, in the sitting posture the sitting angle is no greater than 130°, preferably no greater than 120° and advantageously no greater than 110° and/or no smaller than 60°, preferably no smaller than 70° and advantageously no smaller than 80°. In particular, the sitting angle is approximately 90° in the sitting posture. In particular, in the partly sitting posture the sitting angle is no greater than 170°, preferably no greater than 160° and advantageously no greater than 150° and/or no smaller than 100°, preferably no smaller than 110° and advantageously no smaller than 120°. In particular, the sitting angle is approximately 130° in the partly sitting posture. Preferably, the sitting angle equals the angle between the thigh and the shank of the person. In particular, the sitting angle is approximately 180° in a standing posture.

In particular, the knee joint is pivotably connecting the lower leg to the upper leg, preferably pivotably about a knee joint axis. Advantageously, the knee joint axis is oriented at least substantially perpendicularly, or perpendicularly, to the upper leg longitudinal axis and/or to the lower leg longitudinal axis. Preferably, the knee joint axis is oriented at least substantially perpendicularly, or perpendicularly, to the sitting direction. Advantageously, the upper leg support and the lower leg support together implement at least a portion of the knee joint, or the knee joint. Preferably, a value of the sitting angle corresponds to a value of a knee joint position of the knee joint. In this context "at least substantially perpendicular" is in particular to be understood as an orientation of a direction with respect to a reference direction, in particular in a plane, wherein the direction and the reference direction include an angle, which angle deviates from an angle of 90° by no more than 15º, advantageously by no more than 10º and particularly advantageously by no more than 2º.

Preferably, the wearable sitting posture assisting device comprises at least one locking unit, which is configured for locking the upper leg with respect to the lower leg and/or the knee joint in a certain sitting angle and/or is configured for defining a smallest sitting angle. Advantageously, the locking unit is configured for locking the knee joint in different sitting angles, and/or for defining different smallest sitting angles, which sitting angles can be preferably chosen by the person. It is conceivable that the locking unit is configured for allowing to increase the sitting angle in a locked state. In particular, the person is enabled to stand up when the locking unit is in the locked state. Preferably, the locking unit is configured for enabling the person to sit down again at the defined smallest sitting angle after standing up with the locking unit still defining the same smallest sitting angle. Advantageously, the locking unit comprises at least one blocking element which is configured for blocking and/or unblocking the knee joint and/or for locking the sitting angle and/or for defining a smallest sitting angle. Preferably, the blocking element is implemented as a spring, in particular as a gas spring. Advantageously, the blocking element is connected to the upper leg, in particular to the upper leg support, and to the lower leg, in particular to the lower leg support. Preferably, the blocking element is configured for damping a movement of the upper leg with respect to the lower leg during sitting down and/or during standing up. Advantageously, the locking unit features at least one actuation element, which is configured for actuating the blocking element. Preferably, the actuation element is configured for enabling the person to block or unblock the blocking element. Advantageously, the actuation element is a mechanical actuation element. It is also conceivable that the actuation element is an electronic actuation element. It is further conceivable that the locking unit comprises at least one control unit which is configured for detecting a sitting down condition when the person is sitting down and/or detecting a standing up condition when the person is standing up and/or trigger the actuation element according to a requirement for a blocking of the blocking element.

In particular, the foot unit is configured for connecting to a shoe and/or to a foot of the person and/or the foot unit is connected to a shoe and/or to a foot of the person. Preferably, the foot unit comprises at least one shoe connector for connecting to the foot and/or to the shoe of the person. Advantageously, the foot connector features at least one shoe strap. In particular, the foot connector features at least one upper strap, which advantageously runs across an instep of the foot or of the shoe the foot unit is connected to. Preferably, the foot connector features at least one lower strap, which advantageously runs across a sole of the foot or of the shoe the foot unit connected to. Preferably, the foot connector is configured for being worn on a shoe and/or on a foot. Advantageously, the foot unit comprises at least one foot unit support. Preferably, the shoe connector is connected to the foot unit support. Advantageously, the foot unit support comprises at least one bracket and/or the foot unit support element is implemented as a bracket. Preferably, the shoe strap, in particular the upper strap and/or the lower strap, is connected to the bracket. It is conceivable that the foot unit support element is at least partly implemented integrally, or implemented integrally, with the lower leg, in particular with the lower leg support.

In particular, the ground contact unit comprises at least one ground contact element. Preferably, the ground contact element features at least one ground contact surface, which is advantageously configured for contacting a ground when the person is sitting or partly sitting on the wearable sitting posture assisting device. Advantageously, the ground contact surface is bent and/or curved, in particular convexly bent and/or convexly curved. Preferably, at least a portion of the ground contact element or the entire ground contact element is ellipsoidally and/or spheroidally and/or spherically shaped. In particular, the ground contact element is at least partly, preferably at least to a large extent, advantageously completely made of rubber. Preferably, a weight force of the person is transmitted from the seat unit to the upper leg support and/or from the upper leg support to the knee joint and/or from the knee joint to the lower leg support and/or from the lower leg support to the ground contact element and/or from the ground contact element to the ground. In particular, the weight of the person is additionally transmitted to the ground via the foot or shoe of the person. Preferably, the ground contact element is arranged on a rear side of the shoe of the person. When the person is sitting or partly sitting on the wearable sitting posture assisting device, the foot and/or the shoe of the person is in contact with the ground in addition to the ground contact element. Preferably, the ground contact element is arranged contactlessly with respect to the ground when the person is walking or standing while wearing the sitting posture assisting device.

In particular, the person is wearing the shoe connector on the shoe and/or on the foot in the shoe connector wearing state. Preferably, the shoe connector is attached to the shoe and/or the foot of the person in the shoe connector wearing state. In particular, in the normal wearing state the shoe connector is in a foot unit wearing state. The foot unit wearing state is in particular different from the shoe connector wearing state. Advantageously, the shoe connector wearing state is a condition in which the person puts the wearable sitting posture assisting device on or off.

Preferably, the shoe connector is movable relative to the lower leg freely in the foot in the connector wearing state. In particular, the shoe connector is movable of a distance of at least 1 cm, preferably of at least 2 cm, further preferably of at least 5 cm, advantageously of at least 10 cm and further preferably of at least 20 cm in the shoe connector wearing state. Advantageously, the shoe connector is movable relative to the lower leg in a direction towards the lower leg and/or in a direction away from the lower leg.

For the purpose of providing a wearable sitting posture assisting device, which can be put on and/or off easily and/or comfortably, it is proposed that the foot unit is connectable to and disconnectable from the lower leg. Preferably, the foot unit is in a disconnected state in the shoe connector wearing state. In particular, the foot unit is in a disconnected state when the person puts on the wearable sitting posture assisting device or when the person puts off the wearable sitting posture assisting device. Preferably, the foot unit is in a connected state in the normal wearing state. In particular, the foot unit is connected to the lower leg in the connected state. Advantageously, a position of the foot unit with respect to the lower leg support is fixed in the connected state. Advantageously, the foot unit is configured for being connected to and disconnected from the lower leg repeatedly and/or without damage.

A high degree of user-friendliness can be achieved and/or a wearable sitting posture assisting device, which can be put on and off in a time-efficient manner, can be provided if the foot unit is connectable to and/or disconnectable from the lower leg without tools. In particular, the foot unit is configured for being disconnected from and/or connected to the lower leg using only one hand. Advantageously, the foot unit is configured for being connected to the lower leg via moving the lower leg towards the foot unit. In particular, the foot unit is configured for being connected to and/or disconnected from the lower leg while the person is standing or sitting on a chair or a bench or a stool or a step or the like, in particular prior to and/or after wearing the wearable sitting posture assisting device.

In a further embodiment of the invention it is proposed that the foot unit is configured for connecting to the lower leg in a connection direction, which connection direction is oriented at least substantially parallel to the lower leg longitudinal axis. Preferably, the lower leg is configured for being slid over a portion of the foot unit and/or the foot unit is configured for being slid over a portion of the lower leg in the connection direction and/or in a direction opposite to the connection direction. Preferably, the connection direction points from the foot unit towards the lower leg. In particular, the connection direction is oriented at least substantially parallel, or parallel, to the ground. Preferably, the connection direction is oriented at least substantially perpendicular, or perpendicular, to the sitting direction. Advantageously, the foot unit is partly arranged within the lower leg, in particular within the lower leg support, in the connected state. As a result, intuitive handling can be achieved.

A wearable sitting posture assisting device, which can be used by users of different size and/or with different body dimensions, can be provided if the foot unit is adjustable with respect to the lower leg to at least one first foot unit position and to at least one second foot unit position. In particular, the first foot unit position is a different position of the foot unit with respect to the lower leg as the second foot unit position. Advantageously, the foot unit can be fixed with respect to the lower leg in the first foot unit position and/or in the second foot unit position.

For the purpose of providing an adjustment mechanism, in particular a size adjustment mechanism, which is easily and/or fast and/or intuitively operable, it is proposed that the first foot unit position and the second foot unit position are arranged along the lower leg longitudinal axis. Preferably, a length of a lower portion of the wearable sitting posture assisting device comprising the lower leg and the foot unit, in particular a length parallel to the lower leg longitudinal axis, is different in case the foot unit is in the first foot unit position and in case the foot unit is in the second foot unit position. Advantageously, the first foot unit position defines a first length of the lower portion. Further advantageously, the second foot unit position defines a second length of the lower portion. In particular, the foot unit is adjustable with respect to the lower leg to at least three, preferably to at least four, further preferably to at least five, advantageously to at least six, further advantageously to at least seven or to even more different foot unit positions. Preferably, the foot unit positions are discrete positions. It is also conceivable that at least some of the foot unit positions or all of the foot unit positions are distributed continuously, in particular along the lower leg longitudinal axis. Preferably, the lower leg defines foot unit positions corresponding to an extra small size and/or to a small size and/or to a medium size and/or to a large size and/or to an extra large size, in particular of the person and/or of a length of the leg and/or of the shank of the person. Preferably, the foot unit positions are configured for adjusting a position of the knee joint with respect to the knee of the person in the normal wearing condition.

For the purpose of reducing a constructional complexity, it is proposed that the first foot unit position corresponds to a first connection position and the second foot unit position corresponds to a second connection position of the foot unit. In particular, the foot unit is connectable to the lower leg in different connection positions. Advantageously, every foot unit position corresponds to one connection position. Preferably, the foot unit is disconnectable from the leg unit from different foot unit positions, in particular from all foot unit positions.

It is further proposed that the wearable sitting posture assisting device comprises at least one fixing unit having at least one fixing element for fixing the foot unit to the lower leg. Advantageously, the foot unit and the lower leg, in particular the lower leg support, together implement the fixing unit. Preferably, the fixing unit is configured for connecting the foot unit to and/or for disconnecting the foot unit from the lower leg. In particular, the fixing unit comprises at least one receiving element for the fixing element. Preferably, the foot unit comprises the fixing element and/or the lower leg comprises the receiving element. It is also conceivable that the lower leg comprises the fixing element and/or that the foot unit comprises the receiving element. Preferably, the receiving element and/or the fixing element correspond to one of the foot unit positions. Advantageously, the fixing unit comprises at least one fixing element per foot unit position and/or at least one receiving element per foot unit position, which fixing elements and/or receiving elements are advantageously associated with the respective foot unit position.

Intuitive operability can be achieved if the fixing unit features at least one push button, which is implemented for actuating the fixing element. Preferably, the push button is configured for actuation with one finger, in particular a thumb. Advantageously, the push button is connected to the fixing element.

For the purpose of providing a secure connection between a lower leg and a foot unit of a wearable sitting posture assisting device, it is proposed that the fixing element is movable in a direction at least substantially perpendicular to the lower leg longitudinal axis into at least one fixing position. It is conceivable that the fixing unit comprises at least one spring element for pushing the fixing element into the fixing position. It is also conceivable that the push button is configured for moving the fixing element into the fixing position.

For the purpose of achieving a high degree of user-friendliness concerning connection and disconnection of parts, it is proposed that the fixing element is implemented as a latching element. Preferably, the fixing element is latched with the receiving element in the connected state. In particular, the receiving element is implemented as a hole. Advantageously, the foot unit snaps to the lower leg during connectiing the foot unit to the lower leg. Advantageously, the fixing unit comprises at least one disabling element for the receiving element. In particular, the disabling element is configured for preventing the fixing element from connecting to the receiving element, in particular from latching with the receiving element. Advantageously, the fixing unit comprises the disabling element for different receiving elements. Preferably, the disabling element is removable from the foot unit and/or from the lower leg. It is also conceivable that the disabling element is connected to the foot unit and/or to the lower leg. Advantageously, a positioning of one or more disabling elements with respect to the receiving elements determines a size of the lower portion.

Advantageous characteristics concerning construction and/or design can be achieved if the foot unit features the foot unit support which the shoe connector is connected to. Preferably, the foot unit support implements the fixing unit together with the lower leg, in particular with the lower leg support. Advantageously, the foot unit support is partly located within the lower leg support in the connected state. Advantageously, the different foot unit positions correspond to different positions of the foot unit support with respect to the lower leg support, in particular to different positions of the foot unit support within the lower leg support, in particular along the lower leg longitudinal axis.

In addition, it is proposed that the shoe connector is connectable to and disconnectable from the foot unit support. In particular, it is conceivable that the foot unit support is permanently connected to the lower leg. Advantageously, in particular in this case, the foot unit support is adjustable to different foot unit positions while being connected to the lower leg. For instance, it is conceivable that the foot unit support is slidably mounted to the lower leg, in particular to the lower leg support. Preferably, the shoe connector is configured for snapping to the foot unit support. Advantageously, the shoe connector is configured for being connected to and/or for being disconnected from the foot unit support, in particular using only one hand.

For the purpose of achieving high wearing comfort, in particular when walking with a wearable sitting posture assisting device, it is proposed that the shoe connector is pivotable about a foot pivot axis, which is oriented at least substantially perpendicular to the lower leg longitudinal axis, in particular in the connected state. Preferably, the foot unit comprises at least one bearing which pivotably connects the shoe connector to the foot unit support. Advantageously, the bearing implements a releasable connection of the shoe connector to the foot unit support.

For the purpose of achieving high wearing comfort, in particular when sitting on a wearable sitting posture assisting device, it is proposed that the shoe connector is pivotable about a shank pivot axis, which is oriented at least substantially parallel to the lower leg longitudinal axis, in particular in the connected state. Preferably, at least a portion of the foot unit support, or the foot unit support, or the entire foot unit, is rotatable about the shank pivot axis with respect to the lower leg.

A high degree of handling comfort can be achieved with a foot unit for a wearable sitting posture assisting device according to the invention.

The invention further encompasses a method for putting on a wearable sitting posture assisting device, comprising at least one leg unit featuring at least one upper leg and at least one lower leg; and comprising at least one foot unit having at least one shoe connector for connecting to a shoe or a foot of a person, wherein the shoe connector is attached to the shoe in a disconnected state of the shoe connector in order to yield at least one shoe connector wearing state.

In particular, the shoe connector is connected to the shoe and/or to the foot of the person in a first method step, in particular by the person. In particular, the entire shoe connector is put on the foot and/or on the shoe of the person in the first method step. Preferably, a foot unit of the additional leg unit is put on an other shoe and/or on an other foot of the person in the first method step. In particular, the person puts on the shoe connectors of the wearable sitting posture assisting device in the first method step.

Advantageously, the lower leg is connected to the shoe connector in a second method step, preferably subsequently to the first method step. Preferably, the foot unit is connected to the lower leg in the second method step. Alternatively or additionally, it is also conceivable that the shoe connector is connected to the support element in the second method step. Preferably, the person proceeds analogously in case of the additional leg unit.

In a third method step the person puts on the upper body wearing unit.

The first method step and/or the second method step and/or the third method step are preferably performed in reverse order when putting off the wearable sitting posture assisting device.

Herein, the wearable sitting posture assisting device and the method according to the invention are not to be limited to the application and implementation described above. In particular, for the purpose of fulfilling a functionality herein described, the wearable sitting posture assisting device and the method according to the invention may comprise a number of respective elements, structural components, units and/or steps that differ from the number mentioned herein. Furthermore, regarding the value ranges mentioned in this disclosure, values within the limits mentioned are to be understood to be also disclosed and to be used as applicable.

### Drawings

Further advantages may become apparent from the following description of the drawing. In the drawing exemplary embodiments of the invention are shown. The drawing, the description and the claims contain a plurality of features in combination. The person having ordinary skill in the art will purposefully also consider the features separately and will find further expedient combinations.

If there is more than one specimen of a certain object, only one of these is given a reference numeral in the figures and in the description. The description of this specimen may be correspondingly transferred to the other specimens of the object.

It is shown in :
- Fig. 1: a person wearing a wearable sitting posture assisting device, in a schematic lateral view,
- Fig. 2: the person wearing the wearable sitting posture assisting device, in a schematic front view,
- Fig. 3: the person wearing the wearable sitting posture assisting device in a schematic rear view,
- Fig. 4: a portion of a lower leg of a leg unit of the wearable sitting posture assisting device and a foot unit of the wearable sitting posture assisting device, in a schematic lateral view,
- Fig. 5: a portion of the lower leg and a portion of the foot unit, in a schematic perspective view,
- Fig. 6: a portion of a fixing unit of the wearable sitting posture assisting device, in a schematic front view,
- Fig. 7: a first alternative disabling element of the fixing unit, in a perspective view,
- Fig. 8: a second alternative disabling element for the fixing unit, in a perspective view,
- Fig. 9: a portion of the fixing unit together with a third alternative disabling element, in a schematic front view,
- Fig. 10: a portion of an alternative fixing unit for the wearable sitting posture assisting device, in a perspective view,
- Fig. 11: a schematic flowchart of a method for putting on the wearable sitting posture assisting device,
- Fig. 12: a portion of a lower leg of a leg unit of a first alternative wearable sitting posture assisting device and a foot unit of the first alternative wearable sitting posture assisting device, in a schematic lateral view,
- Fig. 13: a portion of the lower leg and a portion of the foot unit of the first alternative wearable sitting posture assisting device, in a schematic perspective view,
- Fig. 14: the foot unit of the first alternative wearable sitting posture assisting device, in a perspective rear view,
- Fig. 15: the foot unit of the first alternative wearable sitting posture assisting device, in a perspective front view,
- Fig. 16: a portion of a fixing unit of the first alternative wearable sitting posture assisting device, in a perspective sectional view,
- Fig. 17: the portion of a fixing unit of the first alternative wearable sitting posture assisting device, in another perspective sectional view,
- Fig. 18: a portion of a fixing unit of a second alternative wearable sitting posture assisting device, in a perspective view,
- Fig. 19: a portion of the fixing unit of the second alternative wearable sitting posture assisting device, in a schematic sectional side view,
- Fig. 20: a fixing element of the fixing unit of the second alternative wearable sitting posture assisting device, in a schematic bottom view,
- Fig. 21: a portion of the fixing element of the second alternative wearable sitting posture assisting device, in a perspective view,
- Fig. 22: a portion of a fixing unit of a third alternative wearable sitting posture assisting device, in a schematic sectional view,
- Fig. 23: a lower portion of a fourth alternative wearable sitting posture assisting device, in a perspective view,
- Fig. 24: a lower portion of a fifth alternative wearable sitting posture assisting device, in a perspective view and
- Fig. 25: a lower portion of a sixth alternative wearable sitting posture assisting device, in a perspective view.

### Description of the exemplary embodiments

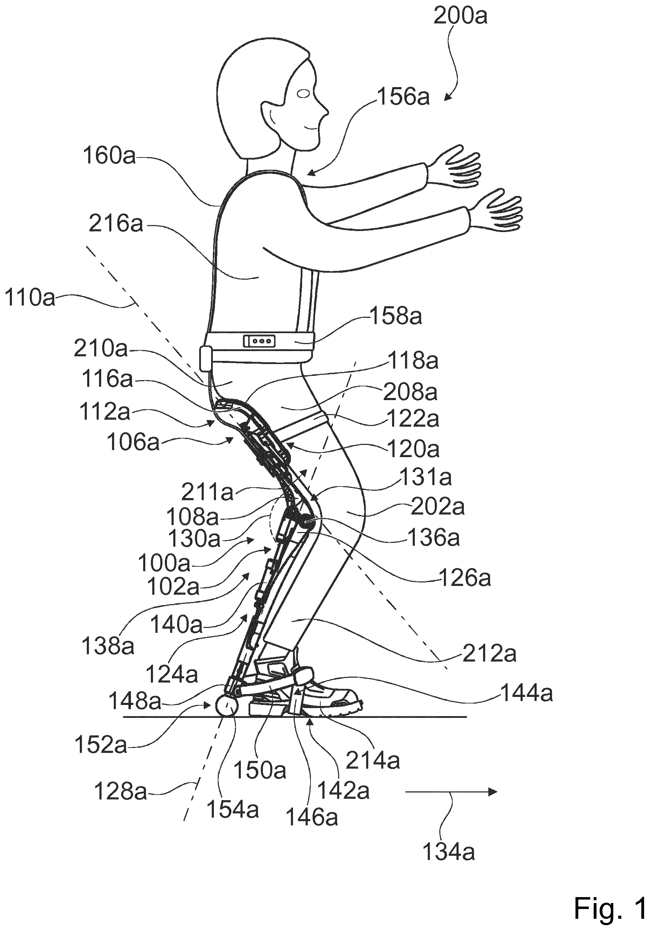

Fig. 1 shows a person 200a wearing a wearable sitting posture assisting device 100a. The wearable sitting posture assisting device 100a is configured for receiving a weight force of the person 200a in a sitting posture or in a partly sitting posture. In fig. 1 the person 200a is shown in a partly sitting posture. In the partly sitting posture a knee 202a of the person is partly bent. In a sitting posture the knee 202a is bent more strongly than in the partly sitting posture. The wearable sitting posture assisting device 100a is configured for allowing the person 200a to sit down on it in different sitting postures and in different partly sitting postures. Furthermore, the wearable sitting posture assisting device 100a is configured for allowing the person 200a to walk while wearing the wearable sitting posture assisting device 100a. In addition, the wearable sitting posture assisting device 100a is configured for allowing the person 200a to stand and/or stand up and/or sit down and/or walk while wearing the wearable sitting posture assisting device 100a.

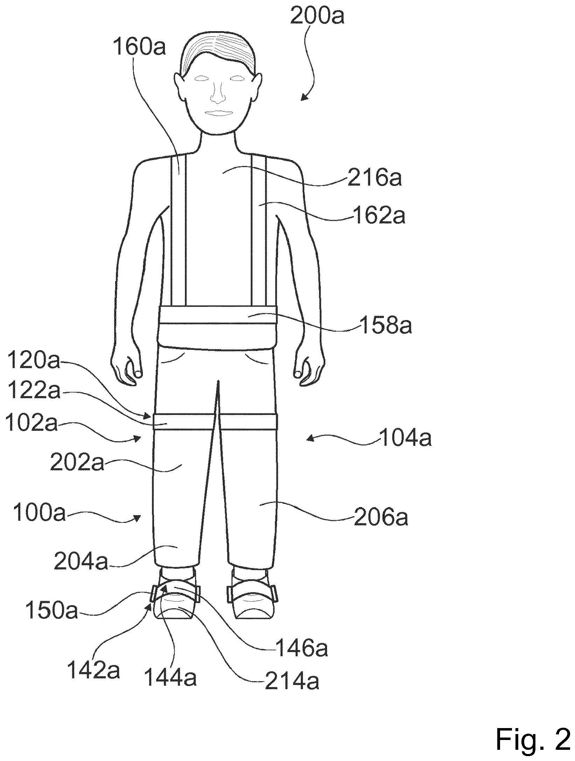

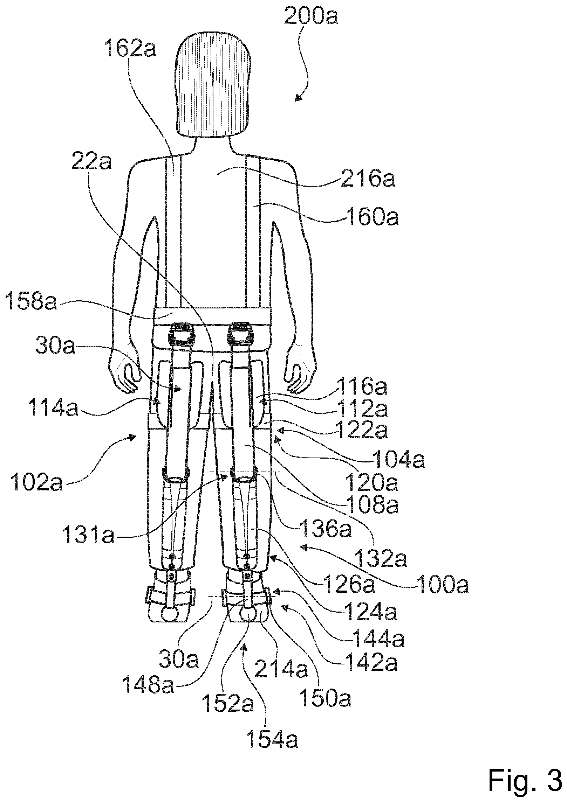

Fig. 2 shows the person 200a wearing the wearable sitting posture assisting device 100a, in a schematic front view. Fig. 3 shows the person 200a wearing the wearable sitting posture assisting device 100a, in a schematic rear view. In figs 1 to 3 the wearable sitting posture assisting device 100a is shown in a normal wearing condition. The normal wearing conditions encompasses a condition in which the person 200a is sitting or partly sitting on the wearable sitting posture assisting device 100a, a condition in which the person 200a is standing up, a condition in which the person 200a is sitting down, a condition in which the person 200a is standing, and a condition in which the person 200a is walking, in each case while wearing the wearable sitting posture assisting device 100a. In the shown case the person 200a is wearing the wearable sitting posture assisting device 100a in a factory building, in particular while working on an assembly line. In a similar fashion it is conceivable that the person 200a wears the wearable sitting posture assisting device 100a in an office building, in a factory building, in a service building, outside, at work, at home, while working, during breaks, etc. Advantageously, the person 200a wears the wearable sitting posture assisting device 100a during an activity which requires the person 200a to sit down and/or to partly sit down and/or to stand up and/or to stand and/or to walk repeatedly. The person 200a can then sit down on the wearable sitting posture assisting device 100a when required, stand up while wearing the wearable sitting posture assisting device 100a when required, and walk while wearing the wearable sitting posture assisting device 100a when required.

The wearable sitting posture assisting device 100a comprises a leg unit 102a. Furthermore, the wearable sitting posture assisting device 100a comprises an additional leg unit 104a. The additional leg unit 104a is implemented identically to the leg unit 102a. Therefore, in the following, only the leg unit 102a is described in detail. The description of the leg unit 102a is to be understood as transferable to the additional leg unit 104a. It is also conceivable that an additional leg unit is implemented mirror-symmetrically to the leg unit. In particular, it is conceivable that a leg unit and an additional leg unit are implemented as right leg unit and left leg unit, respectively, or vice versa.

In the case shown, the person 200a is wearing the leg unit 102a on a right leg 204a. The leg unit 102a is arranged on a rear side 211 a of the leg 204a of the person 200a. Furthermore, the person 200a is wearing the additional leg unit 104a on a left leg 206a. It is also conceivable that a person wears a leg unit on a left leg and an additional leg unit on a right leg. Furthermore, it is conceivable that a person only wears one leg unit. In addition, it is conceivable that a wearable sitting posture assisting device comprises only one leg unit. It is also conceivable that a leg unit is arranged on a lateral side of a leg and/or on a front side of a leg and/or between two legs of a person.

The person 200a is sitting or partly sitting on the wearable sitting posture assisting device 100a in a sitting direction 134a. The person 200a faces and/or looks in the sitting direction 134a when facing forward. The sitting direction 134a is oriented parallel to a ground on which the person 200a is sitting or walking or standing.

The leg unit 102a comprises an upper leg 106a. The upper leg 106a comprises an upper leg support 108a. The upper leg 106a has an upper leg longitudinal axis 110a. The upper leg longitudinal axis 110a is oriented perpendicularly to the sitting direction 134a. The upper leg support 108a has a main extension direction which is oriented parallel to the upper leg longitudinal axis 110a. The upper leg longitudinal axis 110a is oriented parallel to a main extension direction of a thigh 208a of the leg 204a of the person, in particular when the person 200a is sitting, and/or partly sitting and/or walking and/or standing up and/or standing while wearing the wearable sitting posture assisting device 100a.

The upper leg 106a comprises a seat unit 112a. The seat unit 112a is connected to the upper leg support 108a. In the partly sitting posture and/or in the sitting posture the person 200a is sitting on the seat unit 112a. In the case shown the person 200a is sitting on the seat unit 112a and on a seat unit 114a of the additional leg unit 104a in the partly sitting posture. The seat unit 112a comprises a sitting element 116a. The sitting element 116a contacts the thigh 208a of the person 200a. Furthermore, in the sitting posture and/or in the partly sitting posture the sitting element 116a contacts a buttock 210a of the person 200a. The seat unit 112a comprises a sitting surface 118a. The sitting element 116a comprises the sitting surface 118a. The sitting surface 118a is configured for allowing the person 200a to sit down on it with the thigh 208a and/or with the buttock 210a. A shape of the sitting surface 118a is at least partly adjusted to the thigh 208a and/or to the buttock 210a of the person 200a. The sitting surface 118a is curved. The sitting surface 118a is concavely curved and/or bent.

It is also conceivable that a wearable sitting posture assisting device comprises only one seat unit, in particular a common seat unit of two leg units. In particular, in this case it is conceivable that the seat unit is saddle-shaped and/or implemented in the manner of a saddle, in particular arranged between the legs of a person.

The upper leg 106a comprises a thigh connection unit 120a for connecting to the thigh 208a of the person 200a. The thigh connection unit 120a is connected to the upper leg support 108a. The thigh connection unit 120a is configured for connecting the upper leg 106a to the thigh 208a of the person 200a. The thigh connection unit 120a comprises a thigh strap 122a. The thigh strap 122a is fixed to the thigh 208a of the person 200a.

The wearable sitting posture assisting device 100a comprises a lower leg 124a. The lower leg 124a comprises a lower leg support 126a. The lower leg 124a has a lower leg longitudinal axis 128a. The lower leg longitudinal axis 128a is oriented perpendicularly to the sitting direction. The lower leg longitudinal axis 128a and the upper leg longitudinal axis 110a are arranged in a common plane. The lower leg support 126a has a main extension direction which is oriented parallel to the lower leg longitudinal axis 128a. The lower leg longitudinal axis 128a is oriented parallel to a main extension direction of a shank 212a of the leg 204a of the person, in particular when the person 200a is sitting, and/or partly sitting and/or walking and/or standing while wearing the wearable sitting posture assisting device 100a.

The upper leg 106a and the lower leg 124a define a sitting angle 130a. The sitting angle 130a is an angle included by the upper leg longitudinal axis 110a and the lower leg longitudinal axis 128a. The sitting angle 130a is similar or identical to an angle between the thigh 208a and the shank 212a of the person 200a. The sitting angle 130a having a value between 60° and 130°, in particular a value of approximately 90°, corresponds to different sitting postures or to at least one sitting posture. The sitting angle 130a having a value between 130° and 170° corresponds to different partly sitting postures. In case the person 200a is standing while wearing the wearable sitting posture assisting device 100a the sitting angle 130a has a value between 160° and 180°, in particular a value of approximately 180°. In case the person 200a is walking while wearing the wearable sitting posture assisting device 100a the sitting angle 130a may significantly differ from 180°, in particular in case the person 200a bends his knee 202a. In the sitting posture and/or in the partly sitting posture and/or when standing the sitting angle 130a and an analogously defined additional sitting angle of the additional leg unit 104a are advantageously identical. However, it is also conceivable that the person 200a is sitting on the wearable sitting posture assisting device 100a in a sitting posture or a partly sitting posture with the sitting angle 130a and the additional sitting angle being differing, in particular by up to 5°, by up to 10°, by up to 15°, by up to 20°, by up to 30°, by up to 40°, or by more. When the person 200a is walking while wearing the wearable sitting posture assisting device 100a the sitting angle 130a and the additional sitting angle may significantly differ, for instance in case the person bends his knees differently.

The leg unit 102a comprises a knee joint 131 a which pivotably connects the upper leg 106a to the lower leg 124a. The knee joint 131 a connects the upper leg 106a to the lower leg 124a pivotably about a knee joint axis 132a. The knee joint axis 132a is oriented perpendicularly with respect to the upper leg longitudinal axis 110a. The knee joint axis 132a is oriented perpendicularly to the lower leg longitudinal axis 128a. The knee joint axis 132a is oriented perpendicularly to the sitting direction 134a. The knee joint 131 a is partly implemented integrally with the upper leg support 108a. The knee joint 131 a is partly implemented integrally with the lower leg support 126a. The knee joint 131 a comprises at least one bearing 136a which connects the upper leg support 108a to the lower leg support 126a.

The leg unit 102a comprises a locking unit 138a which is configured for locking the knee joint 131 a. The locking unit 138a is configured for limiting the sitting angle 130a to a minimum value. The locking unit 138a is configured for allowing the person 200a to choose the minimum value of the sitting angle 130a. In case the locking unit 138a is in a locked state the person 200a can sit down on the wearable sitting posture assisting device 100a with the sitting angle 130a having the minimum value. The locking unit 138a is configured for being actuated by the person 200a. The locking unit 138a comprises a blocking element 140a. The blocking element 140a is a spring, in particular a gas spring. The blocking element 140a is configured for being locked at different lengths. The blocking element 140a is connected to the upper leg support 108a. The blocking element 140a is connected to the lower leg support 126a. The blocking element 140a is configured for damping a movement of the upper leg 106a with respect to the lower leg 124a, in particular when the person 200a is sitting down.

The leg unit 102a comprises a foot unit 142a. The foot unit 142a is configured for connecting to a shoe 214a and/or to a foot of the person 200a. The foot unit 142a comprises a shoe connector 144a for connecting to the shoe 214a and/or to the foot of the person 200a. The shoe connector 144a comprises a strap 146a which is fixed to the shoe 214a of the person 200a. The foot unit 142a comprises a foot unit support 148a. The foot unit support 148a is connected to the lower leg 124a. The foot unit support 148a is connected to the lower leg support 126a. The foot unit support 148a comprises a bracket 150a. The shoe connector 144a is connected to the foot unit support 148a. The shoe connector 144a is connected to the bracket 150a. The strap 146a is connected to the bracket 150a.

The leg unit 102a comprises a ground contact unit 152a. The ground contact unit 152a is connected to the foot unit 142a. The ground contact unit 152a is connected to the lower leg support 126a. The ground contact unit 152a comprises a ground contact element 154a. When the person 200a is sitting or partly sitting on the wearable sitting posture assisting device 100a the ground contact unit 152a, in particular the ground contact element 154a, is in contact with the ground. The ground contact unit 152a, in particular the ground contact element 154a, is configured for transmitting a part of the weight force of the person 200a into the ground. The ground contact element 154a is rounded. The ground contact element 154a is spherical. The ground contact element 154a is made of rubber. However, other shapes and/or materials are conceivable for a ground contact element as mentioned above.

In case the person 200a is sitting or partly sitting on the wearable sitting posture assisting device 100a the weight force of the person 200a is at least partly, in particular directly or indirectly, transmitted from the seat unit 112a to the upper leg support 108a; from the upper leg support 108a to the knee joint 131 a; from the knee joint 131 a to the lower leg support 126a; from the lower leg support 126a to the ground contact element 154a; from the ground contact element 154a to the ground.

In particular, the weight force of the person 200a is additionally transmitted to the ground via the foot or shoe 214a of the person 200a. Preferably, the ground contact element 154a is arranged on a rear of the shoe 214a of the person 200a. When the person 200a is sitting or partly sitting on the wearable sitting posture assisting device 100a the foot and/or the shoe 214a of the person 200a is in contact with the ground in addition to the ground contact element 154a. Preferably, the ground contact element 154a is arranged contactlessly with respect to the ground when the person 200a is walking and/or standing while wearing the wearable sitting posture assisting device 100a.

The wearable sitting posture assisting device 100a comprises an upper body wearing unit 156a. The person 200a is wearing the upper body wearing unit 156a on his upper body 216a, which upper body 216a may include hips and/or a waist of the person 200a. The upper body wearing unit 156a comprises a belt 158a. Furthermore, the upper body wearing unit 156a comprises suspenders 160a, 162a. The leg unit 102a is connected to the upper body wearing unit 156a. The additional leg unit 104a is connected to the upper body wearing unit 156a. It is conceivable that an upper body wearing unit comprises no belt and only suspenders, or vice versa. It is also conceivable that a wearable sitting posture assisting device is only connected to the legs and/or the feet and/or the shoes of a person wearing it.

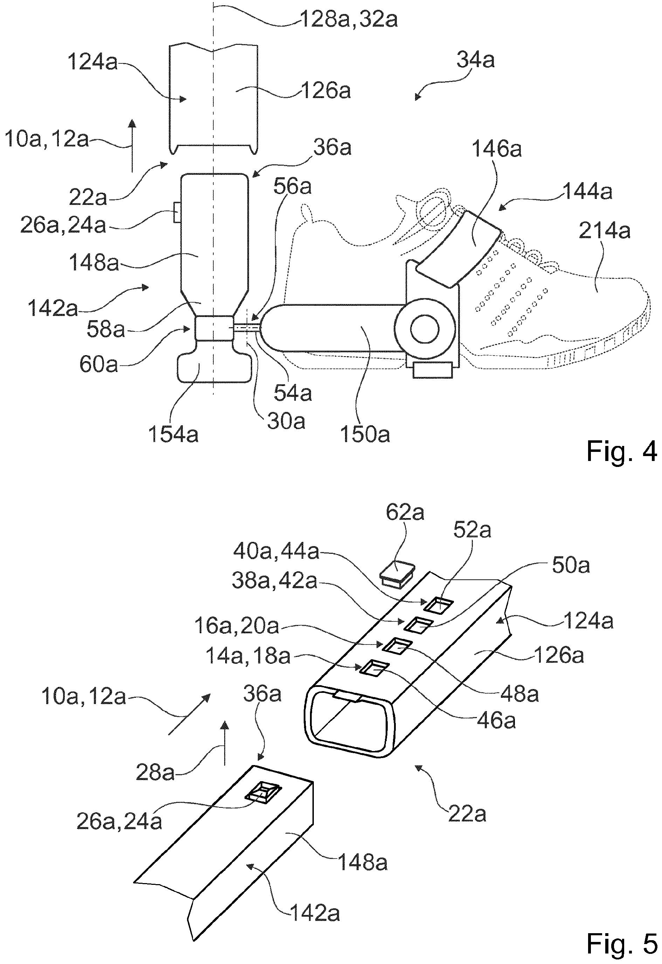

Fig. 4 shows a portion of the lower leg 124a and the foot unit 142a, in a schematic lateral view. Fig. 5 shows a portion of the lower leg 124a and a portion of the foot unit 142a, in a schematic perspective view. The leg unit 102a comprises a lower portion 34a which features the foot unit 142a and a portion of the lower leg 124a. In figs. 4 and 5 the shoe connector 144a is shown in a shoe connector wearing state. In the shoe connector wearing state the shoe connector 144a is attached to the shoe 214a of the person 200a. The person 200a is wearing the shoe connector 144a on his shoe 214a in the shoe connector wearing state. The person 200a is not wearing the wearable sitting posture assisting device 100a in the shoe connector wearing state. In the shoe connector wearing state the shoe connector 144a is disconnected from the lower leg 124a.

The shoe connector 144a is movable relative to the lower leg 124a in a direction 10a parallel to the lower leg longitudinal axis 128a in the shoe connector wearing state. In the case shown the shoe connector 144a is freely movable with respect to the lower leg 124a in the shoe connector wearing state.

The foot unit 142a is connectable to and disconnectable from the lower leg 124a. In the normal wearing condition the foot unit 142a is connected to the lower leg 124a. In the normal wearing condition the shoe connector 144a is connected to the lower leg 124a. In the normal wearing condition the foot unit 142a is in a connected state.

The shoe connector 144a is pivotable about a foot pivot axis 30a, which foot pivot axis 30a is oriented perpendicularly to the lower leg longitudinal axis 128a, in particular in the connected state (compare also fig. 3). The foot unit support 148a implements a pivot mount 56a for the shoe connector 144a about the foot pivot axis 30a. The foot unit support 148a comprises a pivot element 54a which implements the pivot mount 56a. The pivot element 54a is bendable. The pivot element 54a is made of plastic. The pivot element 54a connects the bracket 150a to a support element 58a of the foot unit support 148a. The pivot element 54a is bendable with respect to the support element 58a of the foot unit support 148a The shoe connector 144a is pivotable about a shank pivot axis 32a, which shank pivot axis 32a is oriented parallel to the lower leg longitudinal axis 128a. The foot unit support 148a implements a pivot mount 60a for the shoe connector 144a about the shank pivot axis 32a. The bracket 150a is pivotable about the shank pivot axis 32a.

The foot unit 142a is connectable to the lower leg 124a without tools. The foot unit 142a is disconnectable from the lower leg 124a without tools. The foot unit 142a is configured for being connected to the lower leg 124a with one hand. The foot unit 142a is configured for being disconnected from the lower leg 124a with one hand.

The foot unit 142a is configured for connecting to the lower leg 124a in a connection direction 12a. The connection direction 12a is oriented at least substantially parallel to the lower leg longitudinal axis 128a. When connecting the foot unit 142a to the lower leg 124a the foot unit is moved with respect to the lower leg 124a in the connection direction 12a. In the case shown the foot unit 142a remains in a fixed position with respect to the ground during connection. Furthermore, the lower leg 124a is moved towards the foot unit 142a in a direction opposite to the connection direction 12a. During connection of the foot unit 142a to the lower leg 124a a portion of the foot unit 142a, in particular a top end 36a of the foot unit support 148a, is slid into the lower leg support 126a. In the connected state, the foot unit 142a is partly arranged within the lower leg support 126a.

The foot unit 142a is adjustable with respect to the lower leg 124a to at least one first foot unit position 14a and to at least one second foot unit position 16a. In the case shown the foot unit 142a is adjustable with respect to the lower leg 124a to four different foot unit positions 14a, 16a, 38a, 40a, namely to the first foot unit position 14a, to the second foot unit position 16a, to a third foot unit position 38a and to a fourth foot unit position 40a.

The first foot unit position 14a and the second foot unit position 16a are arranged along the lower leg longitudinal axis 128a. In the case shown all foot unit positions 14a, 16a, 38a, 40a are arranged along the lower leg longitudinal axis 128a. The foot unit positions 14a, 16a, 38a, 40a are arranged equidistantly.

The first foot unit position 14a corresponds to a first connection position 18a of the foot unit 142a. The second foot unit position 16a corresponds to a second connection position 20a of the foot unit 142a. The third foot unit position 38a corresponds to a third connection position 42a of the foot unit 142a. The fourth foot unit position 40a corresponds to a fourth connection position 44a of the foot unit 142a. In the case shown each foot unit position 14a, 16a, 38a, 40a corresponds, in particular biuniquely, to one of the connection positions 18a, 20a, 42a, 44a.

The foot unit 142a is connectable to the lower leg support 126a in the different connection positions 18a, 20a, 42a, 44a. Connecting the foot unit 142a to the lower leg support 126a in different connection positions 18a, 20a, 42a, 44a results in different lengths of the lower portion 34a, in particular parallel to the lower leg longitudinal axis 128. In the case shown the first connection position 18a corresponds to a large size. Furthermore, the second connection position 20a corresponds to a medium size. Furthermore, the third connection position 42a corresponds to a small size. Furthermore, the fourth connection position 44a corresponds to an extra small size.

The wearable sitting posture assisting device 100a comprises a fixing unit 22a. The fixing unit 22a comprises a fixing element 24a for fixing the foot unit 142a to the lower leg 124a. The fixing element 24a is configured for fixing the foot unit 142a to the lower leg 124a, in particular to the lower leg support 126a, in the connected state. The foot unit 142a comprises the fixing element 24a.

The fixing element 24a is implemented as a latching element. The fixing unit 22a comprises a receiving element 46a for receiving the fixing element 24a in the connected state. The receiving element 46a implements the first foot unit position 14a. The receiving element 46a implements the first connection position 18a. The receiving element 46a is implemented as a hole. The fixing unit 22a comprises a second receiving element 48a, a third receiving element 50a and a fourth receiving element 52a. The receiving elements 46a, 48a, 50a, 52a each implement one of the foot unit positions 14a, 16a, 38a, 40a, in particular biuniquely. The receiving elements 46a, 48a, 50a, 52a each implement one of the connection positions 18a, 20a, 42a, 44a, in particular biuniquely. The lower leg support 126a comprises the receiving element 46a. The lower leg support 126a comprises all receiving elements 46a, 48a, 50a, 52a.

In the exemplary embodiment a total of four different foot unit positions 14a, 16a, 38a, 40a, a total of four connection positions 18a, 20a, 42a, 44a, and a total of four receiving elements 46a, 48a, 50a, 52a is shown. The number of four is to be understood a purely exemplary. Other numbers of foot unit positions and/or connection positions and/or receiving elements are conceivable, for instance two, or three, or five, or six, or seven, or eight, or nine, or ten, or even more. It is further conceivable that foot unit positions and/or connection positions are continuously distributed, in particular along the lower leg longitudinal axis. In particular in this case a fixing element may be configured for fixing a foot unit in arbitrary positions within a certain interval, for instance via at least one friction connection between the foot unit and a lower leg.

The fixing unit 22a features a push button 26a, which is implemented for actuating the fixing element 24a. In the case shown the fixing element 24a is identical with the push button 26a. The fixing element 24a is implemented as a push button 26a. The push button 26a is movable with respect to the foot unit support 148a.

The fixing element 24a is movable in a direction 28a at least substantially perpendicular to the lower leg longitudinal axis 128a into at least one fixing position. In fig. 5 the fixing element 24a is shown in the fixing position. The fixing element 24a is configured for being pushed into the foot unit support 148a, in particular upon actuation of the push button 26a, in particular out of the fixing position and into a retracted position, in which the foot unit support 148a can be inserted into the lower leg support 126a in order to connect the foot unit 142a to the lower leg 124a. The fixing element 24a is spring-mounted to the lower leg support 126a. The fixing element 24a is spring-mounted to the lower leg support 126a in a way that the fixing element 24a is automatically restored into the fixing position. The fixing element 24a is spring-mounted to the lower leg support 126a in a way that it counteracts a pushing force exerted onto the push button 26a, which pushing force pushes the fixing element 24a at least partly into the lower leg support 126a.

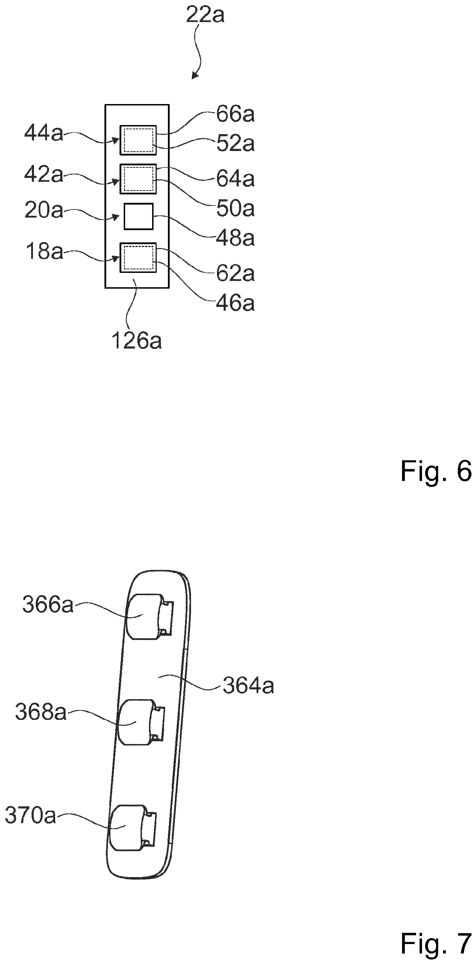

Fig. 6 shows a portion of the fixing unit 22a, in a schematic front view. The fixing unit 22a comprises a disabling element 62a for the receiving element 46a. The disabling element 62a is configured for being used with any of the receiving elements 46a, 48a, 50a, 52a. The disabling element 62a is configured for disabling the receiving element 46a. The disabling element 62a is configured for disabling a foot unit position 14a, 16a, 38a, 40a and/or a connection position 18a, 20a, 42a, 44a. The disabling element 62a is configured for being placed in the receiving element 46a. The disabling element 62a is configured for blocking the receiving element 46a. The fixing element 24a latches only with receiving elements 46a, 48a, 50a, 52a that are unblocked. The disabling element 62a is configured for preventing latching of the fixing element 24a to a receiving element 46a, 48a, 50a, 52a in which it is placed. The disabling element 62a jams the receiving element 46a. The disabling element 62a is made of rubber. A disabling element might also be made of plastic.

In the case shown in fig. 6 the fixing unit 22a comprises three disabling elements 62a, 64a, 66a. The disabling elements 62a, 64a, 66a disable three receiving elements 46a, 50a, 52a. Thus, the disabling elements 62a, 64a, 66a disable three connection positions 18a, 42a, 44a. In the case shown the second connection position 20a is not disabled by a disabling element. When connecting the foot unit 142a to the lower leg support 126a the fixing element 24a latches with the second receiving element 48a. The lower portion 34a is therefore set to a medium size. A size of the lower portion 34a and/or of the wearable sitting posture assisting device 100a is adjustable depending on a positioning of disabling elements 62a, 64a, 66a. For the case shown the disabling elements 64a, 66a could be omitted. When sliding the foot unit support 148a into the lower leg support 126a the fixing element 22a would pass the receiving element 46a since it is blocked by the disabling element 62a. After passing the first receiving element 46a, the fixing element 22a would latch with the second receiving element 48a in the second connection position 20a of the foot unit 142a.

In particular for the purpose of achieving a high degree of safety when connecting the foot unit 142a to the lower leg 124a the topmost receiving element 52a may always be unblocked, in particular not disabled by a disabling element. Thus, at least one receiving element 46a, 48a, 50a, 52a may always be available for the fixing element 24b. As a result, in such a case the foot unit 142a will always snap to the lower leg 124b when the lower leg support 126a is slid over the foot unit support 148a. The foot unit 142a can be advantageously prevented from falling off of the lower leg 124a.

Fig. 7 shows a first alternative disabling element 364a for the fixing unit 22a, in a perspective view. The first alternative disabling element 364a comprises three protrusion elements 366a, 368a, 370a. The protrusion elements 366a, 368a, 370a are configured for disabling three adjacent connection positions 18a, 20a, 42a, 44a, in particular analogously to the disabling element 62a of fig. 6. In the case shown the first alternative disabling element 364a could be used for setting the lower portion 34a to a large size if positioned in the upper three receiving elements 48a, 50a, 52a or to a extra small size if positioned in the lower three receiving elements 46a, 48a, 50a.

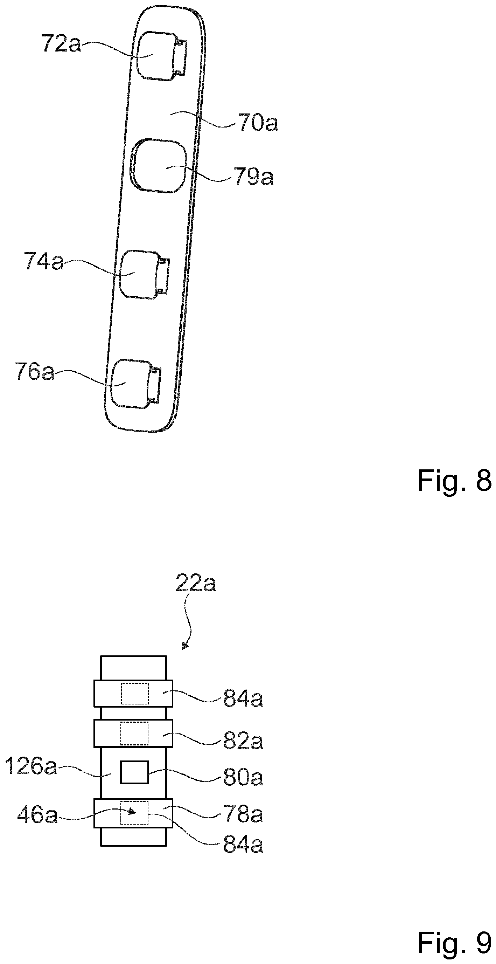

Fig. 8 shows a second alternative disabling element 70a for the fixing unit 22a, in a perspective view. The second alternative disabling element 70a comprises three protrusion elements 72a, 74a, 76a, which are configured for disabling three connection positions 18a, 20a, 44a. The disabling element 70a comprises an access opening 79a through which one receiving element 50a is accessible. The respective connection position 42a is therefore usable when using the second alternative disabling element 70a. The other receiving elements 46a, 50a, 52a are blocked by the second alternative disabling element 70a. In the case shown the second alternative disabling element 70a could be used for setting the lower portion 34a to a medium size, or to a small size if the second alternative disabling element 70a was used upside down.

It is conceivable that a disabling element implemented analogously to the first alternative disabling element 64a of fig. 6 and/or implemented analogously to the second alternative disabling element 70a of fig. 7 comprises a different number and/or arrangement of protrusion elements and/or a different number and/or arrangement of access openings, in particular in order to define a connection position in which the foot unit 142a is connected to the lower leg unit 124a.

Fig. 9 shows a portion of the fixing unit 22a together with a third alternative disabling element 78a, in a schematic front view. The third alternative disabling element 78a is implemented as a rubber band. The third alternative disabling element 78a comprises a protrusion element 80a which is configured for blocking a receiving element 46a, 48a, 50a, 52. In the case shown the protrusion element 80a blocks the first receiving element 46a. Furthermore, in the case shown the fixing unit 22a comprises three third alternative disabling elements 78a, 82a, 84a.

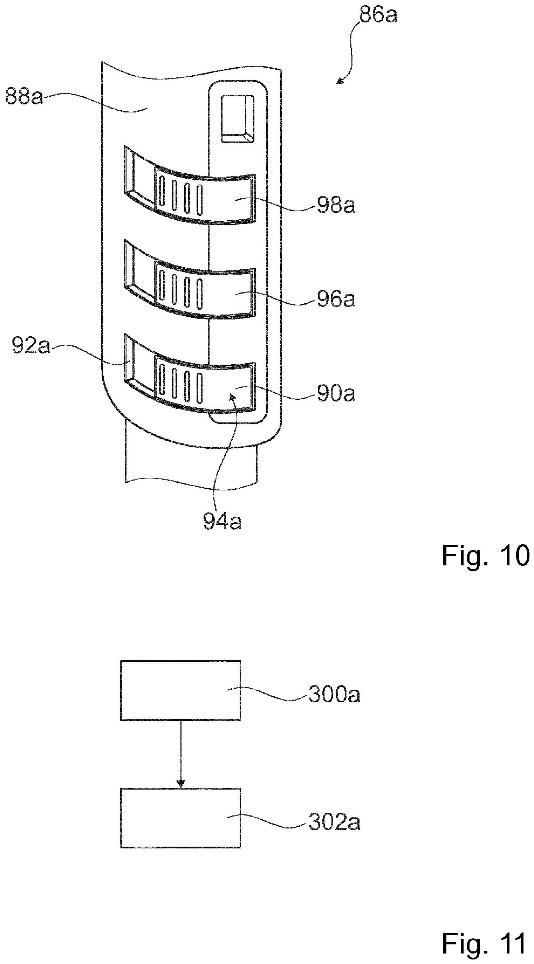

Fig. 10 shows a portion of an alternative fixing unit 86a for the wearable sitting posture assisting device 100a, in a perspective view. The alternative fixing unit 86a is implemented analogously to the fixing unit 22a. The alternative fixing unit 86a is partly implemented by an an alternative lower leg support 88a. The alternative lower leg support 88a is implemented analogously to the lower leg support 126a. The following description is substantially limited to the differences between the alternative fixing unit 86a and the fixing unit 22a, and to the differences between the alternative lower leg support 88a and the lower leg support 126a.

The fixing unit 86a comprises a disabling element 90a. The disabling element 90a is implemented as a slider. The disabling element 90a is movably mounted in a guide slot 92a. The disabling element 90a is movable with one finger. The disabling element 90a is configured for snapping into a disabling position and into an enabling position. In fig. 10 the disabling element 90a is shown in the disabling position. In the enabling position the disabling element 90a uncovers a receiving element 94a of the alternative fixing unit 86a.

In the case shown the alternative fixing unit 86a comprises a plurality of disabling elements 90a, 96a, 98a, which are implemented identically. The positioning of the disabling elements 90a, 96a, 98a allows the person 200a to set a size of the wearable sitting posture assisting device 100a.

Fig. 11 shows a schematic flowchart of a method for putting on the wearable sitting posture assisting device 100a. In a first method step 300a the shoe connector 144a is attached to the shoe 214a of the person 200a, in particular by the person 200a, in the disconnected state of the shoe connector 144a in order to yield the shoe connector wearing state. In the case shown the foot unit 142a is in the disconnected state in the first method step 300a. In a second method step 302a the foot unit 142a is connected to the lower leg 124a. The foot unit 142a is connected to the lower leg 124a in the second method step 302a without tools, in particular by the person 200a.

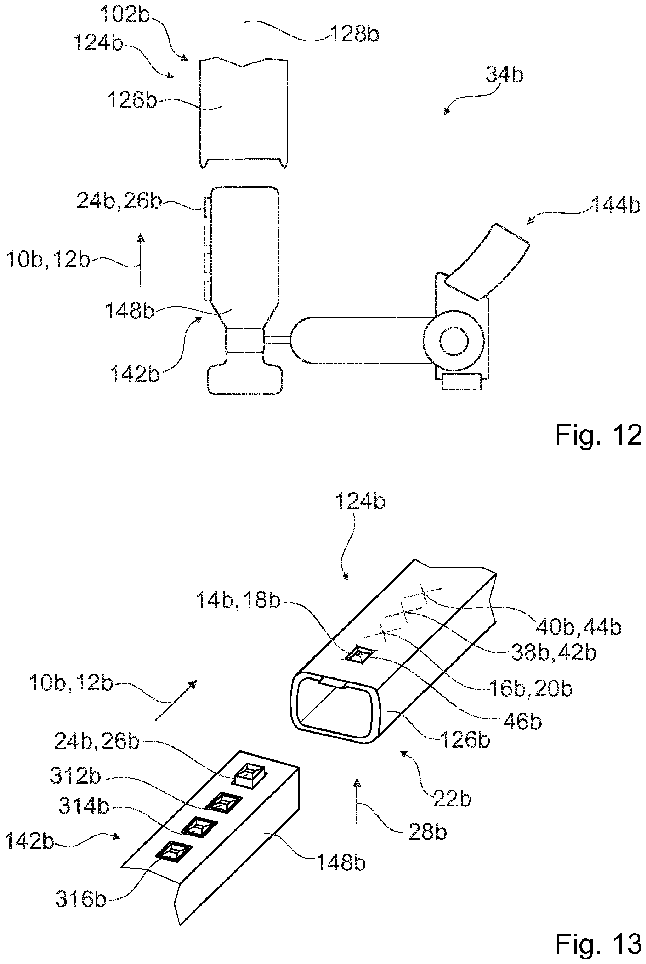

Figs. 12 to 23 show further exemplary embodiments of the invention. The following description is substantially limited to the differences between the exemplary embodiments, wherein regarding structural elements, features and functions that remain the same the description of the other exemplary embodiments, in particular the exemplary embodiment of figs. 1 to 11, may be referred to. For distinguishing the exemplary embodiments, the letter a of the reference numerals in the exemplary embodiment of figs. 1 to 11 has been substituted by the letters b to g in the reference numerals of the exemplary embodiments of figs. 12 to 23. Regarding structural elements having the same denomination, in particular regarding structural elements having the same reference numerals, principally the drawing and/or the description of the other exemplary embodiments, in particular of the exemplary embodiment of figs. 1 to 11, may be referred to.

Fig. 12 shows a lower portion 34b of a leg unit 102b of a first alternative wearable sitting posture assisting device, in a schematic lateral view. The lower portion 34b comprises a portion of a lower leg 124b of the leg unit 102b. The lower leg 124b defines a lower leg longitudinal axis 128b. The lower leg 124b comprises a lower leg support 126b. Furthermore, the lower portion 34b comprises a foot unit 142b of the first alternative wearable sitting posture assisting device. The foot unit 142b comprises a shoe connector 144b. The foot unit 142b comprises a foot unit support 148b.

The shoe connector 144b is movable relative to the lower leg 124b in a direction 10b parallel to the lower leg longitudinal axis 128b in a shoe connector wearing state. The foot unit 142b is connectable to and disconnectable from the lower leg 124b.

The foot unit 142b is configured for connecting to the lower leg 124b in a connection direction 12b, which connection direction 12b is oriented parallel to the lower leg longitudinal direction 128b.

The foot unit 142b is adjustable with respect to the lower leg 124b to at least one first foot unit position 14b and to at least one second foot unit position 16b. In addition, the foot unit 142b is adjustable with respect to the lower leg 124b to a third foot unit position 38b and to a fourth foot unit position 40b. The foot unit positions 14b, 16b, 38b, 40b are arranged along the lower leg longitudinal axis 128b. The foot unit positions 14b, 16b, 38b, 40b correspond to connection positions 18b, 20b, 42b, 44b of the foot unit 142b.

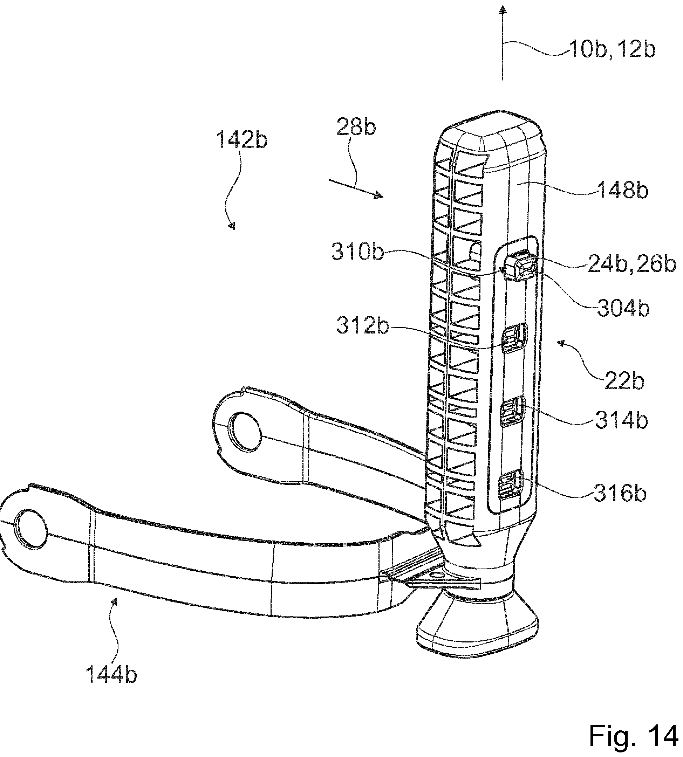

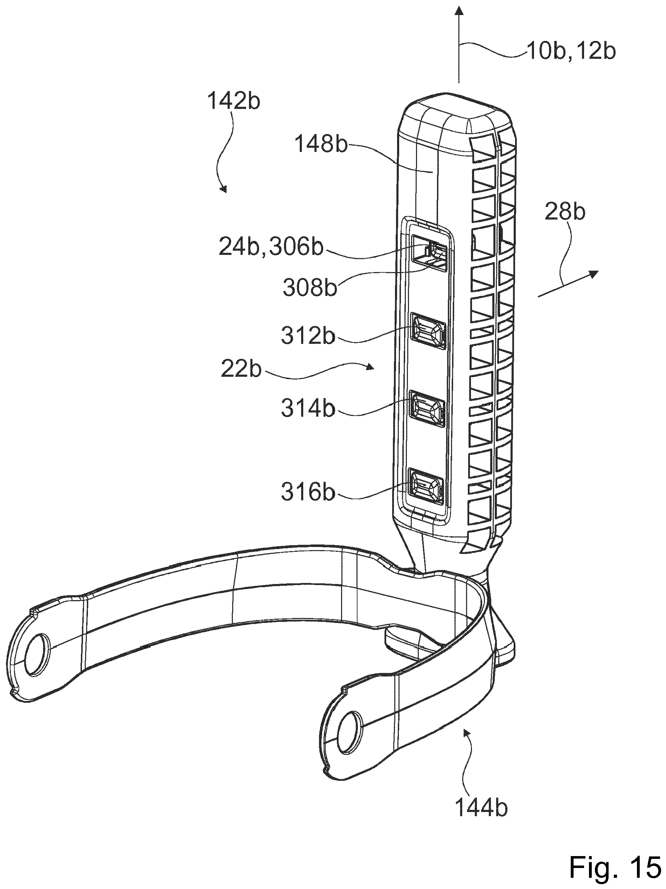

Fig. 13 shows a portion of the lower portion 34b, in a schematic perspective view. Fig. 14 shows the foot unit 142b, in a perspective rear view. Fig. 15 shows the foot unit 142b, in a perspective front view. In fig. 14 and 15 the shoe connector 144b is only partly shown for reasons of clarity. The wearable sitting posture assisting device comprises a fixing unit 22b. The foot unit 142b and the lower leg 124b together implement the fixing unit 22b. The fixing unit 22b comprises a fixing element 24b for fixing the foot unit 142b to the lower leg 124b. The foot unit 142b comprises the fixing element 24b.

The fixing unit 22b comprises a push button 26a which is implemented for actuating the fixing element 24b. The fixing element 24b implements the push button 26b. The push button 26b is implemented by a surface 304b of the fixing element 24b. The fixing element 24b can be moved by pushing onto the surface 304b. The push button 26b implements an additional push button 306b. The fixing element 24b comprises an additional surface 308b which implements the additional push button 306b. The additional surface 308b is arranged opposite the surface 304b.

The fixing element 24b is movable in a direction 28b perpendicular to the lower leg longitudinal axis 128b into at least one fixing position. The fixing element 24b is shown in the fixing position in figs. 12 to 15. The fixing element 24b is movable into the fixing position via pushing onto the additional surface 308b. The fixing element 24b is movable from the fixing position into a disabled position via pushing onto the surface 304b. The fixing element 24b is configured for latching in the fixing position. The fixing element 24b is configured for latching in the disabled position.

The lower leg support 126b comprises a receiving element 46b for the fixing element 24b. The receiving element 46b is implemented as a hole. In a connected state of the foot unit 142b the fixing element 24b is latched with the receiving element. The fixing element 24b comprises a spring-mounted portion 310b which can be pushed into the lower leg support 126b during a sliding of the foot unit support 148b into the lower leg support 126b. The spring-mounted portion 310b is configured for latching with the receiving element 46b. Additionally or alternatively, it is also conceivable that a lower leg support comprises a spring-mounted receiving element.

In the case shown the fixing unit 22b comprises three additional fixing elements 312b, 314b, 316b. The fixing elements 312b, 314b, 316b are implemented identically to the fixing element 24b. The fixing elements 24b, 312b, 314b, 316b are, in particular equidistantly, arranged along the lower leg longitudinal axis 128b. The fixing elements 24b, 312b, 314b, 316b are configured for defining the connection positions 18b, 20b, 42b, 44b. Each of the fixing elements 24b, 312b, 314b, 316b corresponds to one size of the lower portion 34b. A size of the first alternative wearable sitting posture assisting device can be adjusted to a desired length by moving, in particular only, the respective fixing element 24b, 312b, 314b, 316b into its fixing position. In the case shown the fixing element 24b is in its fixing position, while the additional fixing elements 312b, 314b, 316b are in their disabled positions. In the case shown the first alternative wearable sitting posture assisting device is adjusted to a large size.

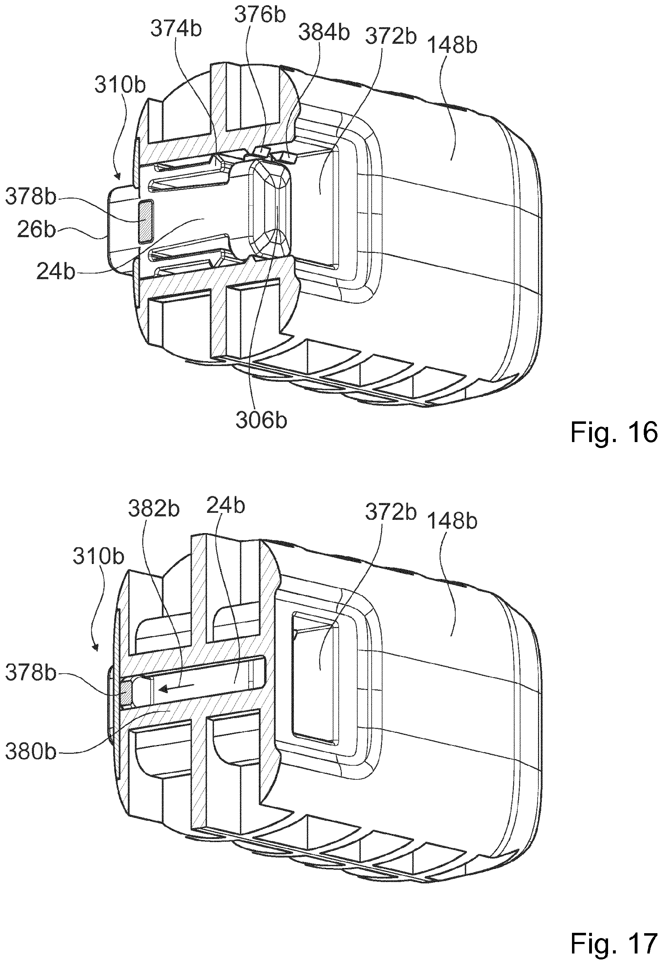

Fig. 16 shows a portion of the fixing unit 22b, in a perspective sectional view. Fig. 17 shows the portion of the fixing unit 22b, in another perspective sectional view. The fixing element 26b is made of one piece. The fixing element 26b is arranged within a bushing 372b of the foot unit support 148b. The fixing element 26b comprises a latching element 374b for latching the fixing element 26b in the disabled position and in the fixing position. The lower leg support 148b comprises a protrusion element 376b. The latching element 374b is pushed past the protrusion element 376b when pushing the push button 26b for disabling the fixing element 24b. The protrusion element 376b implements a guiding for the fixing element 24b together with a second protrusion element 384b.

The fixing element 24b comprises a protruding element 378b which is slidable within a guide track 380b of the bushing 372b. A spring element 382b is arranged within the guide track 380b. The spring element 382b is schematically shown as an arrow indicating a pushing direction of the spring element 382b. The spring element 382b is configured for generating a pushing force onto the spring-mounted portion 310 of the fixing element 24b. The spring element 382b pushes the fixing element 26b into the fixing position, in particular in case the latching element 374b is not latched with the protrusion element 376b and/or in particular in case the fixing element 374b is not in the disabled position. When sliding the lower leg support 126b over the foot unit support 148b the fixing element 26b is pushed into the bushing 372b against the spring element 382b. Subsequently, the fixing element 26b is pushed into the receiving element 46b. The fixing element 26b fixes the foot unit support 148b to the lower leg support 126b. The fixing element 26b latches with the lower leg support 148b.

Furthermore, the spring element 382b pushes the fixing element 26b into the fixing position when the push button 306b is pushed.

In particular for the purpose of achieving a high degree of safety when connecting the foot unit 142b to the lower leg 124b the fixing element 316b may be permanently in its fixing position. Thus, at least one fixing element 24b, 312b, 314b, 316b may always be in a fixing position. As a result, in such a case the foot unit 142b will always snap to the lower leg 124b when the lower leg support 126b is slid over the foot unit support 148b. The foot unit 142b can be advantageously prevented from falling off of the lower leg 124b.

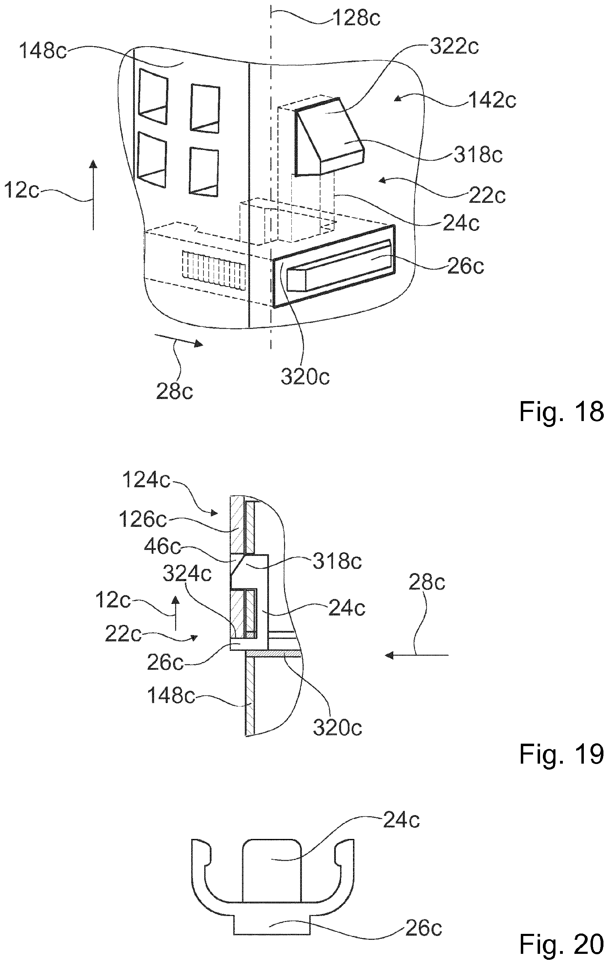

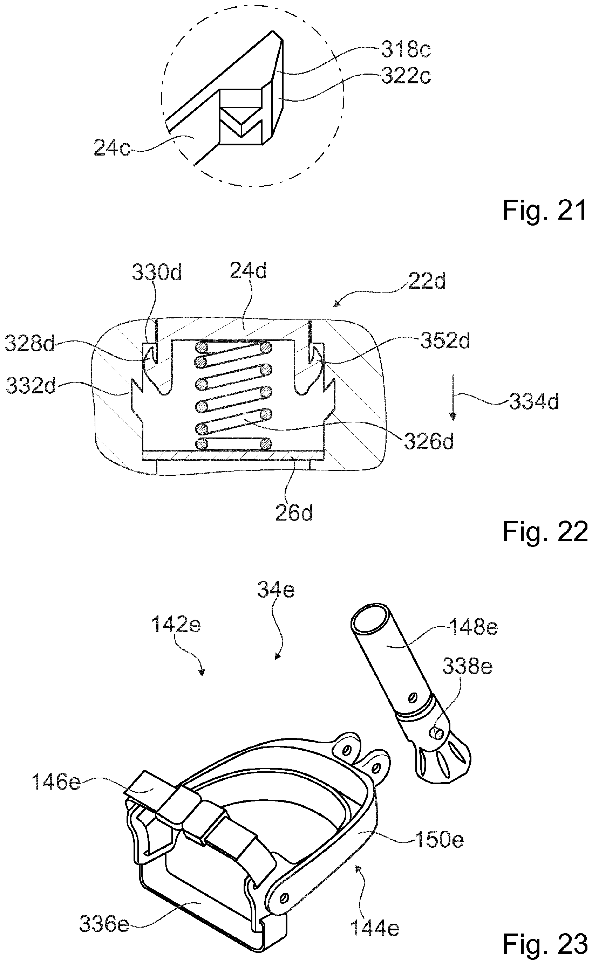

Fig. 18 shows a portion of a fixing unit 22c of a second alternative wearable sitting posture assisting device, in a perspective view. Fig. 19 shows a portion of the fixing unit 22c, in a schematic sectional side view. Fig. 20 shows a fixing element 24c of the fixing unit 22c, in a schematic bottom view. Fig. 21 shows a portion of the fixing element 24c, in a perspective view.

The second alternative wearable sitting posture assisting device comprises a foot unit 142c featuring a foot unit support 148c. The second alternative sitting posture assisting device comprises a lower leg 124c featuring a lower leg support 126c. The lower leg support 126c defines a lower leg longitudinal axis 128c. The foot unit 142b is connectable to and disconnectable from the lower leg 124c without tools. The foot unit 142c is configured for connecting to the lower leg 124c in a connection direction 12c. The connection direction 12c is oriented parallel to the lower leg longitudinal axis 128c.

The second alternative wearable sitting posture assisting device comprises a fixing unit 22c. The fixing unit 22c comprises a fixing element 24c for fixing the foot unit 142c to the lower leg 124c. The fixing element 24c comprises a latching element 318c. The fixing unit 22c features a push button 26c which is implemented for actuating the fixing element 24c, in particular the latching element 318c. The fixing element 24c implements the push button 26c. The fixing element 24c is made of one piece.

The fixing unit 22c comprises a guiding element 320c which sliding-mounts the fixing element 24c with respect to the lower leg support 126c. The guiding element 320c is arranged within the lower leg support 126c. The fixing element 24c is movable in a direction 28c perpendicular to the lower leg longitudinal axis into a fixing position. The fixing element 24c is spring-mounted in a way that the spring element is automatically pushed into the fixing position. The latching element 318c comprises a pushed surface 322c, which is oriented inclined with respect to the lower leg longitudinal axis 128c. The pushed surface 322c slides on the lower leg support 126c during connection of the foot unit 142c to the lower leg 124c. The lower leg support 126c comprises a receiving element 46c for the latching element 318c.

In a connected state of the foot unit 142c the latching element 318c is latched with the receiving element 46c. The latching element 318c secures the foot unit 142c against being pulled out of the lower leg support 126c in the connected state. Furthermore, a lower end 324c of the lower leg support 126c touches the push button 26c in the connected state. The push button 26c secures the foot unit 142c against being pushed further into the lower leg support 126c. The fixing element 24c and the receiving element 46c are configured for fixing a position of the foot unit 142c relative to the lower leg 124c.

The foot unit 142c can be disconnected from the lower leg 124c via pushing the push button 26c and pulling the foot unit 142c out of the lower leg support 126c.

Fig. 22 shows a portion of a fixing unit 22d of a third alternative wearable sitting posture assisting device, in a schematic sectional view. The fixing unit 22d comprises a fixing element 24d, which is only partly shown in fig. 22. The fixing element 24d is movable from a disabled position into a fixing position, and vice versa. In fig. 20 the fixing element 24d is shown in the fixing position. The fixing unit 22d comprises a push button 26d for actuating the fixing element 24d. The fixing unit 22d comprises a spring 326d. The spring 326d connects the fixing element 24d to the push button 26d. The fixing element 24d comprises a latching element 328d. The latching element 328d is made of a deformable material. The latching element 328d is made of a flexible material. The latching element 328d is made of rubber. It is also conceivable that a latching element is made of plastic and/or metal.

The fixing unit 22d comprises a first receiving element 330d for the latching element 328d and a second receiving element 332d for the latching element 328d. In the fixing position the latching element 328d is latched with the first receiving element 330d. In case the latching element 328d is pushed in a direction 334d towards the push button 26d the latching element 328d latches with the second receiving element 332d. The latching element 328d is latched with the second receiving element 332d in the disabled position. In case the push button 26d is pushed when the fixing element 24d is in the disabled position, the latching element 328d is pushed out of the second receiving element 332d. The fixing element 24d is then pushed into the fixing position by the spring 326d. The latching element 328d is deformed when the push button 26d is pushed.

The fixing element 24d comprises an additional latching element 352d. The additional latching element 325d is arranged opposite the latching element 328d. The additional latching element 352d is implemented identically to the latching element 328d.

Fig. 23 shows a lower portion 34e of a fourth alternative wearable sitting posture assisting device, in a perspective view. The lower portion 34e comprises a foot unit 142e. The foot unit 142e comprises a foot unit support 148e. The foot unit 142e comprises a shoe connector 144e. The shoe connector 144e comprises a bracket 150e. The bracket 150e is U-shaped. The bracket 150e encompasses a shoe of a person wearing the third alternative wearable sitting posture assisting device from a rear side. Furthermore, the shoe connector 144e comprises a strap 146e. The strap 146e is implemented as an upper shoe strap. The shoe connector 144e comprises an additional strap 336e which is implemented as a lower shoe strap.

The shoe connector 144e is connectable to and disconnectable from the foot unit support 148e. The foot unit support 148e comprises a spring-mounted pin element 338e. The bracket 150e snaps to the pin element 338e when connecting the shoe connector 144e to the foot unit support 148e.

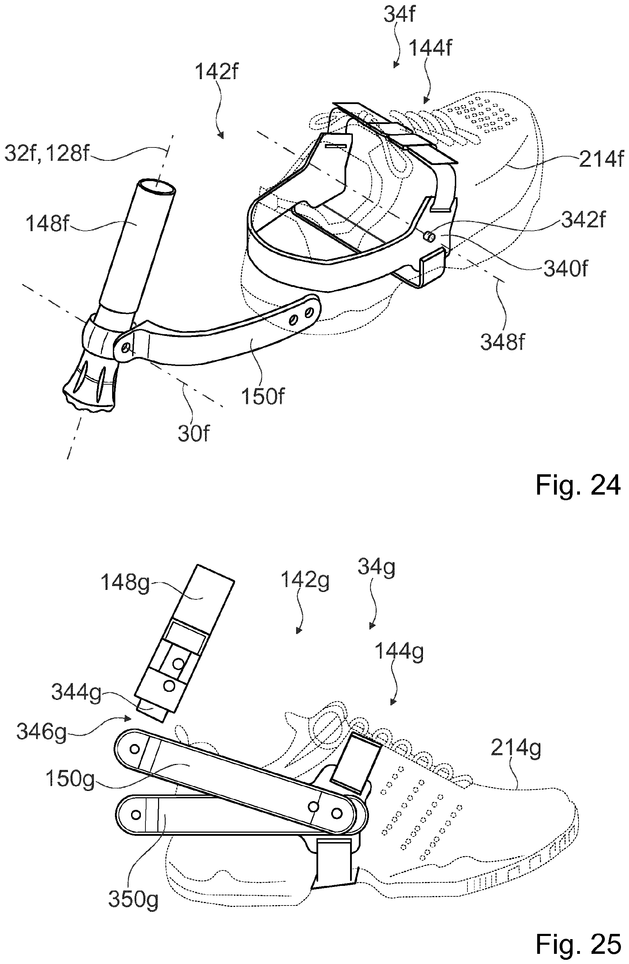

Fig. 24 shows a lower portion 34f of a fifth alternative wearable sitting posture assisting device, in a perspective view. The lower portion 34f comprises a foot unit 142f. The foot unit 142f comprises a foot unit support 148f. The foot unit support comprises a single bracket 150f. The single bracket 150f is one-sided. The single bracket 150f is arranged on a lateral side of a shoe 214f of a person wearing the fifth alternative wearable sitting posture assisting device.

The foot unit 142f comprises a shoe connector 144f. The shoe connector 144f comprises an inner bracket 340f. The inner bracket 340f is made of plastic. The inner bracket 340f is deformable. The inner bracket 340f is flexible. The inner bracket 340f is U-shaped. The inner bracket 340f encompasses the shoe 214f from a rear side. The inner bracket 340f comprises a connection element 342f for connecting to the single bracket 150f. The connection element 342f is implemented as a quick-connection element.

The shoe connector 144f is pivotable about a foot pivot axis 30f, which foot pivot axis 30f is oriented perpendicular to a lower leg longitudinal axis 128f of a lower leg of the fifth alternative wearable sitting posture assisting device. The shoe connector 144f is pivotable about a shank pivot axis 32f, which shank pivot axis 32f is oriented parallel to the lower leg longitudinal axis 128f. In the case shown the shoe connector 144f is also pivotable about a shoe connector pivot axis 348f. The shoe connector pivot axis 348f is oriented parallel to the foot pivot axis 30f. The shoe connector pivot axis 348f runs through the connection element in a connected state of the shoe connector 144f to the foot unit support 148f.

Fig. 25 shows a lower portion 34 g of a sixth alternative wearable sitting posture assisting device, in a perspective view. The lower portion 34g comprises a foot unit 142g. The foot unit 142 g comprises a foot unit support 148g.

The foot unit 142g comprises a shoe connector 144g. The shoe connector 144g is connectable to and disconnectable from the foot unit support 148g. The shoe connector 144g comprises a bracket 150g. The bracket 150g is connectable to a lower tip 344g of the foot unit support 148g. The bracket 150g and the lower tip 344g together implement a quick connection 346g. In the case shown the quick connection 346g is implemented mechanically. Alternatively or additionally, a quick connection 346g may be implemented magnetically.

The sixth alternative wearable sitting posture assisting device is free of a ground contact unit. In particular, the sixth alternative wearable sitting posture assisting device does not comprise a ground contact element. When a person wearing the sixth alternative wearable sitting posture assisting device is sitting on the sixth alternative wearable sitting posture assisting device, a weight force of the person is transmitted to the ground only via the shoes 214g of the person. The weight force of the person is transmitted via the foot unit 142g to a shoe 214g of the person. The shoe connector 144g comprises a support bracket 350g. In a sitting posture or in a partly sitting posture the weight force is transmitted from the foot unit support 148g to the bracket 150g and from the bracket 150g to the support bracket 350g.

## Claims

1. Wearable sitting posture assisting device (100a), comprising at least one leg unit (102a; 102b) featuring at least one upper leg (106a) and at least one lower leg (124a; 124b; 124c), the lower leg (124a; 124b; 124c) defining at least one lower leg longitudinal axis (128a; 128b; 128c; 128f); and comprising at least one foot unit (142a; 142b; 142c; 142e; 142f; 142g) having at least one shoe connector (144a; 144b; 144e; 144f; 144g) for connecting to a shoe (214a; 214f; 214g) or a foot of a person (200a), **characterized in that** the shoe connector (144a; 144b; 144e; 144f; 144g) is movable relative to the lower leg (124a; 124b; 124c) in a direction (10a; 10b) at least substantially parallel to the lower leg longitudinal axis (128a; 128b; 128c; 128f) in at least one shoe connector wearing state.

2. Wearable sitting posture assisting device according to claim 1, **characterized in that** the foot unit (142a; 142b; 142c) is connectable to and disconnectable from the lower leg (124a; 124b; 124c).

3. Wearable sitting posture assisting device according to claim 1 or 2, **characterized in that** the foot unit (142a; 142b; 142c) is connectable to and/or disconnectable from the lower leg (124a; 124b; 124c) without tools.

4. Wearable sitting posture assisting device according to any one of the preceding claims, **characterized in that** the foot unit (142a; 142b; 142c) is configured for connecting to the lower leg (124a; 124b; 124c) in a connection direction (12a; 12b; 12c), which connection direction (12a; 12b; 12c) is oriented at least substantially parallel to the lower leg longitudinal axis (128a; 128b; 128c).

5. Wearable sitting posture assisting device according to one of the preceding claims, **characterized in that** the foot unit (142a; 142b) is adjustable with respect to the lower leg (124a; 124b) to at least one first foot unit position (14a; 14b) and to at least one second foot unit position (16a; 16b).

6. Wearable sitting posture assisting device according to claim 5, **characterized in that** the first foot unit position (14a; 14b) and the second foot unit position (16a; 16b) are arranged along the lower leg longitudinal axis (128a; 128b).

7. Wearable sitting posture assisting device at least according to claims 2 and 5, **characterized in that** the first foot unit position (14a; 14b) corresponds to a first connection position (18a; 18b) and the second foot unit position (16a; 16b) corresponds to a second connection position (20a; 20b) of the foot unit (142a; 142b).

8. Wearable sitting posture assisting device according to one of the preceding claims, **characterized by** at least one fixing unit (22a; 22b; 22c) having at least one fixing element (24a; 24b; 24c) for fixing the foot unit (142a; 142b; 142c) to the lower leg (124a; 124b; 124c).

9. Wearable sitting posture assisting device according to claim 8, **characterized in that** the fixing unit (22a; 22b; 22c; 22d) features at least one push button (26a; 26b; 26c, 26d) which is implemented for actuating the fixing element (24a; 24b; 24c; 24d).

10. Wearable sitting posture assisting device according to claim 8 or 9, **characterized in that** the fixing element (24a; 24b; 24c) is movable in a direction (28a; 28b; 28c) at least substantially perpendicular to the lower leg longitudinal axis (128a; 128b; 128c) into at least one fixing position.

11. Wearable sitting posture assisting device according to one of claims 8 to 10, **characterized in that** the fixing element (24a; 24b; 24c) is implemented as a latching element.

12. Wearable sitting posture assisting device according to one of the preceding claims, **characterized in that** the foot unit (142a; 142e; 142f; 142g) features at least one foot unit support (148a; 148e; 148f; 148g) to which foot unit support (148a; 148e; 148f; 148g) the shoe connector (144a; 144e; 144f; 144g) is connected.

13. Wearable sitting posture assisting device according to claim 12, **characterized in that** the shoe connector (144e; 144f; 144g) is connectable to and disconnectable from the foot unit support (148e; 148f; 148g).

14. Wearable sitting posture assisting device according to one of the preceding claims, **characterized in that** the shoe connector (144a; 144f) is pivotable about a foot pivot axis (30a; 30f), which foot pivot axis (30a; 30f) is oriented at least substantially perpendicularly to the lower leg longitudinal axis (128a; 128f).

15. Wearable sitting posture assisting device according to one of the preceding claims, **characterized in that** the shoe connector (144a; 144f) is pivotable about a shank pivot axis (32a; 32f), which shank pivot axis (32a; 32f) is oriented at least substantially parallel to the lower leg longitudinal axis (128a; 128f).

16. Foot unit (142a; 142b; 142e; 142f; 142g) for a wearable sitting posture assisting device (100a) according to one of the preceding claims.

17. Method for putting on a wearable sitting posture assisting device (100a), in particular according to one of the preceding claims, comprising at least one leg unit (102a) featuring at least one upper leg (106a) and at least one lower leg (124a); and comprising at least one foot unit (142a) having at least one shoe connector (144a) for connecting to a shoe (214a) or a foot of a person (200a), wherein the shoe connector (144a) is attached to the shoe (214a) in a disconnected state of the shoe connector (144a) in order to yield at least one shoe connector wearing state.
